Europäisches Patentamt

European Patent Office

Office européen des brevets

(11) Veröffentlichungsnummer: **0 165 551**
**A2**

# (12) EUROPÄISCHE PATENTANMELDUNG

(21) Anmeldenummer: 85107257.9

(22) Anmeldetag: 12.06.85

(51) Int. Cl.⁴: **G 01 N 33/53**

(30) Priorität: 19.06.84 DE 3422616

(43) Veröffentlichungstag der Anmeldung:
27.12.85 Patentblatt 85/52

(84) Benannte Vertragsstaaten:
AT BE CH DE FR GB IT LI LU NL SE

(71) Anmelder: BEHRINGWERKE Aktiengesellschaft
Postfach 1140
D-3550 Marburg 1(DE)

(72) Erfinder: Gronski, Peter, Dr.
Kirchgraben 5
D-3550 Marburg(DE)

(72) Erfinder: Kranz, Theodor, Dr.
Oberer Eichweg 8
D-3550 Marburg(DE)

(72) Erfinder: Fink, Hans-Joachim, Dr.
Kegelbahn 48
D-6230 Frankfurt am Main 80(DE)

(72) Erfinder: Lappe, Franz, Dr.
Brandenburger Weg 20
D-6238 Hofheim am Taunus(DE)

(72) Erfinder: Magerkurth, Karl-Otto
Weissdornweg 2
D-6238 Hofheim am Taunus(DE)

(72) Erfinder: Post, Lothar,
Berliner Ring 2
D-6233 Kelkheim (Taunus)(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) Verfahren zur Bestimmung eines Partners einer Reaktion und Vorrichtung dazu.

(57) Es werden ein optisches Verfahren zum Nachweis und zur Bestimmung eines Partners einer Reaktion, die unter Agglutination und Präzipitatbildung verläuft sowie eine dazu geeignete Vorrichtung, welche die unmittelbare Vermessung von Präzipitatflächen erlaubt, beschrieben. Verfahren und Vorrichtung sind beispielsweise zur Bestimmung von einem Reaktanden einer immunologischen Reaktion geeignet.

Croydon Printing Company Ltd

## Verfahren zur Bestimmung eines Partners einer Reaktion und Vorrichtung dazu

Die Erfindung betrifft ein optisches Verfahren zum Nachweis und zur Bestimmung eines Partners einer Reaktion, die unter Agglutination und Präzipitatbildung verläuft sowie eine dazu geeignete Vorrichtung.

Für serologische Fragestellungen werden Analyseverfahren angewendet, bei denen die Reaktionspartner den verschiedenen Phasen einer Suspension angehören. So kann es unter bestimmten physikochemischen Bedingungen, wie zum Beispiel der Mehrbindigkeit des gelösten Reaktanden, zu charakteristisch verlaufenden Partikelpräzipitationen (beispielsweise Agglutinationen) kommen. Dabei stellt gewöhnlich eine Teilstruktur einer Blutzellmembran (direkte Agglutination) oder eine zum Beispiel chemisch an diese Membran gebundene, ursprünglich nicht vorhandene Gruppierung (indirekte Agglutination) den Reaktionspartner der festen Phase dar, während ein mit dieser Teilstruktur oder Gruppierung spezifisch reagierender Antikörper sich in der gelösten Phase befindet. Die Phasenverteilung kann auch umgekehrt sein.

Die Agglutination von sehr vielen kleinen Teilchen wie Blutzellen oder Latex-Partikeln, die in serologischen Untersuchungen üblicherweise eingesetzt werden, führt zu Veränderungen der Suspension, die in einem Ansatz von etwa 200 µl auf planen Objektträgern visuell erkannt und halbquantitativ bewertet werden können.

In anderen Fällen führt eine Reaktion von Blutzellen mit gelösten Reaktanden zur Auflösung der Zellen, so daß eine visuelle Auswertung ebenfalls möglich ist.

Anstelle der planen Objektträger, auf denen zwei bis drei Ansätze nebeneinander angesetzt und ausgewertet werden können, setzt man seit einiger Zeit Mikrotitrationsplatten ein, in denen Vertiefungen zur Aufnahme der Reaktionsansätze enthalten sind. In solchen Platten lassen sich eine Vielzahl von Proben bearbeiten. Mikrotitrationsplatten erlauben auch Bestimmungen in mehreren Verdünnungsstufen, so daß die qualitative Bewertung in einer Ja/Nein-Aussage "halbquantitativ" in Form von "Titerstufen" erfolgen kann.

Je nach dem experimentellen Verfahren ist die Form der mit konkavem Boden versehenen Reaktionsgefäße zu wählen: Flache, kugelsegmentförmige Vertiefungen erlauben die Durchmischung der Reaktionsvolumen durch leicht taumelnde Bewegungen der Platte. Bei tieferen Näpfchen mit kegeligem oder halbkugelförmigem Boden werden die Platten nach intensiver Durchmischung unbewegt stehen gelassen.

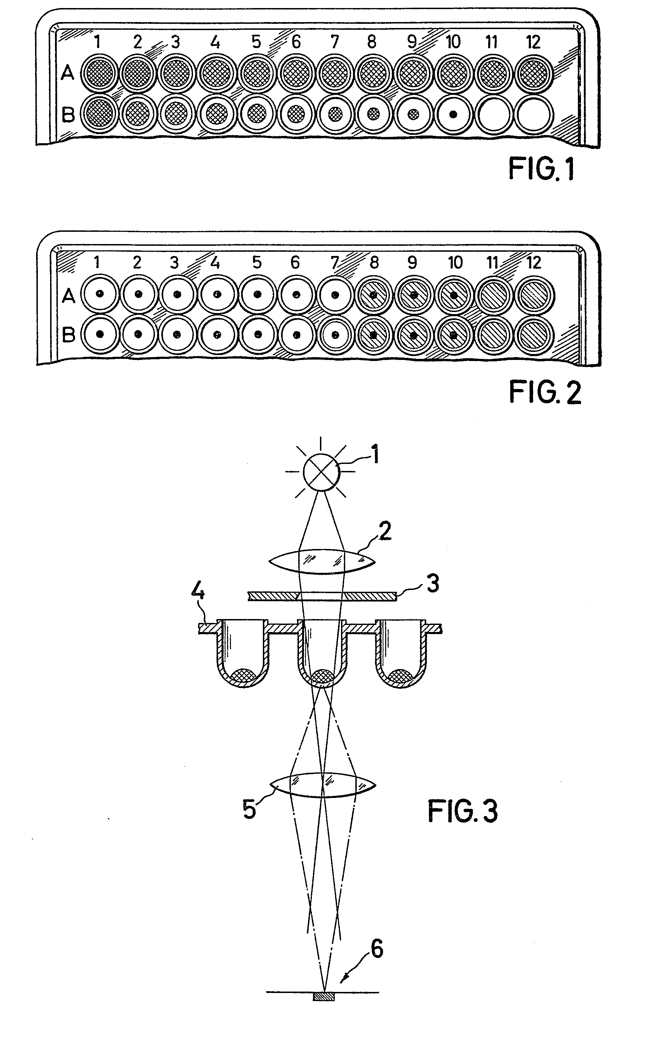

Zur visuellen Auswertung werden die "Muster" ("pattern") der sedimentierten Blutzellen oder Latex-Teilchen vor einem hellen Untergrund bewertet. Nach subjektiven Kriterien wird aus einem Bereich, in dem ein Wechsel des Musters sichtbar ist (Figur 1), eine Verdünnung als "Titerstufe" bestimmt, in der auf Grund der graduellen Ausprägung des bewerteten Kriteriums ein relativer Gehalt des gelösten Reaktanden bestimmt werden kann. Die Genauigkeit derartiger Auswertungen beträgt ± eine Titerstufe, wenn man die Volumen-Reproduzierbarkeit der eingesetzten Verdünnungsgeräte außer Acht läßt und die Auswertungen von einer Person vorgenommen werden.

Die Muster der sedimentierten Teilchen unterscheiden sich je nach Typ der verwendeten Mikrotitrationsplatten; dementsprechend sind auch die Bewertungskriterien verschieden: Während in den Lysistesten (Figur 2) die Menge der noch intakten Zellen bewertet und als Ergebnis eine Verdünnung ausgeprägter Knopfbildung und vollständiger Lysis bestimmt wird, bilden sich bei Agglutinationsansätzen (Figur 1) besondere Muster aus, die als Zellrasen (bei kompletter Agglutination), Ringbildung oder Knopfbildung (bei fehlendem Reaktanden der Agglutinationsreaktion) bewertet werden können.

Erläuterungen zu den Abbildungen:

Figur 1: Indirekte Hämagglutination in einer Mikrotitrationsplatte (an Erythrozyten gebundenes Tetanustoxoid (TT)/humane Anti-TT-IgG-Antikörper).

    a) Die Reihe A (Näpfchen 1 bis 12) zeigt eine vollständige Agglutination.

    b) Die Reihe B zeigt das Ergebnis einer Agglutination, wobei die Antikörperkonzentration von Näpfchen 1 bis 10 linear abnimmt.

Figur 2: Hämolyseassay in einer Mikrotitrationsplatte. In der Reihe A und B wurden 2 Lösungen, die eine unterschiedliche Menge an Immunkomplexen enthielten, in von Näpfchen 1 bis 12 abnehmender Konzentration jeweils mit einer konstanten Menge an menschlichem Serum (Komplementquelle) inkubiert (37°C, 1 h). Anschließend wurde pro Näpfchen eine definierte Menge an Erythrozyten und einer Anti-Erythrozyten-Antikörper-Lösung zugesetzt und stehengelassen (0,5 h, 37°C). Bei ausreichend geringer Immunkomplexkonzentration bewirkt die verbleibende Restkomplementaktivität eine unvollständige (Reihe A, Näpfchen 8 bis 10; Reihe B, Näpfchen 7 bis 10) bzw. vollständige (Reihe A und B, Näpfchen 11 und 12; Lysis der Erythrozyten. In der Reihe A, Näpfchen 1 bis 7, bzw. in der Reihe B, Näpfchen 1 bis 6, ist keine Lysis zu beobachten, da das gesamte Komplement verbraucht worden ist.

Die Auswertung von Lysistesten und Agglutinationsansätzen in Mikrotitrationsplatten mit kugeligen Boden erfolgt weitgehend visuell nach diesen Kriterien, wobei die Präzision (Richtigkeit) und Reproduzierbarkeit (Genauigkeit) auf Grund subjektiver Fehler und starker methodischer Streuung außerhalb der üblicherweise für quantitative Methoden akzeptablen Bereiche liegen.

In Verfahren mit bekannten Vorrichtungen zum Nachweis der Serotypen (JP-A- 123909-79, DOS DE-A- 3033870) werden zur Unterscheidung einer "positiven" und "negativen" Reaktion Röhrchen mit speziell geformtem Boden benutzt, wodurch unterschiedliche "Agglutinationsmuster" entstehen: Fehlt die spezifische Reaktion zwischen Blutzelle und Antikörper, entsteht durch Zusammenrutschen der Zellen in einen vertieften Teil des Röhrchens das sogenannte "Sammelmuster", während nach positiver Reaktion das "Agglutinationsmuster" entsteht, wobei ein Zusammenrutschen infolge Agglutination, das heißt Vernetzung der Blutzellen untereinander, verhindert wird.

Die Messung erfolgt in der bekannten Vorrichtung selbsttätig, indem der Photostrom eines Detektors bei punktförmiger Abtastung des Röhrchenbodens "entlang einem Durchmesser" typische Kurven auf einem Schreiber ergibt: Bei Agglutination ergibt sich eine Kurve mit nur geringer Lichtschwächung im mittleren Bereich des Röhrchenbodens, während bei "negativer" Reaktion die Kumulation der zusammengerutschten Zellen zu einer stärkeren
Lichtschwächung führt. Die auf diese Weise nur qualitative Bewertung (Ja-Nein-Bewertung) kann bei "teilweiser
Agglutination (Halb-Sammelmuster)" graduell nur grob in
einer Stufe differenziert werden.

Die Erfindung hat sich daher die Aufgabe gestellt, ein
Verfahren zur quantitativen Auswertung von Agglutinationsmustern, insbesondere solcher von immunologischen
Reaktionen, zur Verfügung zu stellen.

Die zur Lösung dieser Aufgabe für quantitative analytische Zwecke erforderliche Präzision kann überraschenderweise durch unmittelbare Integration der in den
Ringen eingeschlossenen Flächen erreicht werden, indem
die Präzipitatmuster abgebildet und die Konturen über
lichtempfindliche Sensoren elektro-optisch erfaßt werden
(Figur 3).

Gegenstand der Erfindung ist daher ein optisches Verfahren zum Nachweis und zur Bestimmung eines Partners
einer Reaktion mittels Agglutination, dadurch gekennzeichnet, daß man die durch die Präzipitate der Agglutinationsreaktion begrenzte Fläche unmittelbar mißt und
daraus Menge oder Aktivität des Partners der immunologischen Reaktion quantitativ bestimmt. Dies ist deshalb
möglich, da die Menge oder Aktivität der Reaktionspartner eine monoton wachsende Funktion der Flächengröße ist.

In diesem Verfahren können vorteilhaft mehrere definierte Verdünnungen des quantitativ zu bestimmenden Reaktionspartners verwendet werden.

Ein Reaktionspartner kann im gelösten Zustand, der
andere im gelösten oder an Partikel gebunden vorliegen.

Weiterhin können an der Reaktion eine oder mehrere aktive Komplementkomponenten beteiligt sein.

In einer bevorzugten Ausführungsform läßt man die Präzipitate sich in einem Gefäß mit konkav gebogenem Boden bilden.

Die Meßvorrichtung für das beschriebene Verfahren kann in einfacher Weise aus einer Lichtquelle mit einer Linse und einem Kreuztisch, auf dem entweder die Mikrotitrationsplatte kontinuierlich durch den Strahlengang oder die optische Vorrichtung Lichtquelle/Linse/Detektor kontinuierlich über die festliegende Mikrotitrationsplatte bewegt wird, bestehen. Die in der Bildebene scharf eingestellten hell-dunkel-Konturen des Sedimentes werden durch lichtempfindliche Detektoren erfaßt und rechnerisch ausgewertet.

Gegenstand der Erfindung ist deshalb weiterhin eine optische Vorrichtung zur quantitativen Bestimmung der Fläche zweidimensionaler Objekte, in folgender Reihenfolge bestehend aus a) einer Lichtquelle (1), b) der beleuchtenden Linse (2), c) einer Lochblende (3), d) dem Objekt (4), e) der abbildenden Linse (5) und f) dem Sensor (6) in der Bildebene, der aus mehreren linear angeordneten Detektoren besteht, wobei entweder das Objekt oder der Sensor senkrecht zur optischen Achse beweglich angeordnet ist (Fig. 3), so daß beim Überfahren die gesamte Fläche des Präzipitates erfaßt wird.

Diese optische Vorrichtung kann vorteilhaft zum quantitativen Vermessen von Agglutinationsmuster, an deren Entstehung beispielsweise Antigenmoleküle, Antikörpermoleküle und gegebenenfalls eine oder mehrere aktive Komplementkomponenten beteiligt sind, verwendet werden.

Diese Vorrichtung erlaubt eine sehr genaue quantitative Auswertung, indem durch den Einsatz vieler Detektoren auf einer Sensorleiste die gesamte Fläche einer Reihe von Ring-Mustern erfaßt wird, die sich in sehr empfindlicher Weise bei vibrationsfreiem Stehen der Inkubationsansätze in Röhrchen mit Halbkugel-förmigem Boden ausbilden. Diese besondere Weise der unmittelbaren Flächenerfassung als primären Meßwert bei der erfindungsgemäßen Vorrichtung unterscheidet sich von einem bekannten Verfahren (BE-PS 1549921, US-PS 4193981, DE-A-2744028), bei dem die Durchmesser der Präzipitatringe aus photographischen Aufnahmen der Mikrotitrationsplatten (s. u.) als Maß der Antigen-oder Antikörper-Konzentration vermessen werden.

Die für quantitativ-analytische Zwecke erforderliche Reproduzierbarkeit des beschriebenen Verfahrens wird einesteils erreicht durch die oben beschriebene meßtechnische Erfassung der Präzipitatflächen. Voraussetzung für eine objektive und vergleichbare Erfassung von Platte zu Platte und von Tag zu Tag ist jedoch ferner die Objektivierbarkeit, wobei ein besonderer Vorteil darin besteht, die Messungen am Objekt, der Mikrotitrationsplatte selbst, vornehmen zu können. Die photometrische Vermessung der Durchmesser am Negativ oder Positiv einer photographischen Aufnahme (BE-PS 1549921) vergrößert die Fehlerstreuung durch unterschiedliche Schwärzung des Photomaterials, die durch Abhängigkeit von Belichtungszeit, Beleuchtung, Konditionen bei der Entwicklung (Badtemperatur, Badkonzentration, Entwicklungszeit) entsteht. Darüber hinaus ist Voraussetzung für eine quantitativ-analytische Methode die Reproduzierbarkeit über längere Zeit, die für Hämagglutinationsverfahren nur bei hinreichender Reproduzierbarkeit der Herstellung der eingesetzten Erythrozyten als wesentlichem Reagenz möglich ist, eine Voraussetzung, die bei anderen beschriebenen Verfahren nicht berücksichtigt ist (BE-PS 1549921).

Das Verfahren und die Vorrichtung, die oben beschrieben sind, ermöglichen zum Beispiel Einflüsse des Komplementsystems auf eine Immunkomplexbildung quantitativ zu erfassen. Dies war mit den bisher gebräuchlichen Partikelpräzipitationen, die in Mikrotitrationsplatten durchgeführt werden, nicht möglich. So kann man beispielsweise Immunkomplexe im Antikörperüberschußbereich aus Tetanustoxoid (TT) und humanen Anti-TT-Antikörpern (ATTA) der Klasse IgG in An-oder Abwesenheit (Komplementinaktivierung durch einstündiges Erhitzen auf 56°C) von funktionell aktivem Komplement durch eine halbstündige Inkubation der Reaktanden bei 37°C erzeugen und den Reaktionsansatz unmittelbar danach gemäß oben beschriebener Methode analysieren.

So wurde überraschenderweise gefunden, daß eine komplementbeeinflußte Immunkomplexbildung die zusätzliche Entstehung agglutinierender Aktivitäten verursachen kann. Damit bietet sich eine neue Möglichkeit an, eine funktionelle Überprüfung einer Komplementaktivität durchzuführen. Somit werden zum Beispiel Informationen zugänglich, die bei der Beurteilung pathologisch veränderter Seren (gewisse Komplementmangelzustände oder -dysfunktionen) eine diagnostische Hilfe sein können. Andererseits besteht natürlich auch die Möglichkeit Antikörperpräparationen hinsichtlich ihrer Fähigkeit im Antigenbindenden Zustand mit dem Komplementsystem wechselzuwirken vergleichend zu untersuchen, ein wichtiges Kriterium zu ihrer funktionellen Beurteilung.

Es ist ebenso vorstellbar, daß Partikelpräzipitationen,
denen keine Antigen-Antikörperreaktion zugrunde liegt,
Sedimentationsmuster erzeugen können, die mit dem Verfahren und der Vorrichtung, wie sie oben beschrieben
sind, ausgewertet werden können.

Die folgenden Beispiele sollen die Erfindung erläutern:

## Beispiel 1

Zunächst werden 475 µl frisches, humanes Serum, 175 µl einer Anti-Tetanustoxoid-IgG-Lösung (PBS, pH = 7.2; 7.14 IE/ml) und 100 µl einer Tetanustoxoid-Lösung (PBS, pH = 7.2; 10 Lf/ml) 1 h bei 37°C inkubiert. Unmittelbar danach werden je 50 µl verschiedener Verdünnungen (PBS, pH = 7.2) des Reaktionsansatzes mit 25 µl einer Tetanustoxoid-beschichteten Hammelerythrozytensuspension (20 ml sedimentierte Erythrozyten pro Liter) nach sorgfältiger Durchmischung 2 h bei Raumtemperatur in den Näpfchen von Mikrotitrationsplatten inkubiert und gemäß oben beschriebenem Verfahren ausgewertet. Das Ergebnis einer Dreifachbestimmung ergab einen Gehalt von 0.232 ± 0.006 IE pro ml Reaktionsansatz.

## Beispiel 2

Anstelle des im Beispiel 1 angeführten 475 µl frischen Humanserums wird das gleiche Volumen von Hitze-inaktiviertem, humanem Serum (1 h, 56°C) bei im übrigen unveränderter Verfahrensweise verwendet. Das Ergebnis einer Dreifachbestimmung ergab einen Gehalt von 0.193 ± 0.003 IE/ml.

Patentansprüche

1. Optisches Verfahren zum Nachweis und zur Bestimmung eines Partners einer Reaktion mittels Agglutination, dadurch gekennzeichnet, daß man die durch die Präzipitate der Agglutinationsreaktion begrenzte Fläche unmittelbar mißt und daraus Menge oder Aktivität des Partners der Reaktion quantitativ bestimmt.

2. Verfahren nach Anspruch 1, dadurch gekennzeichnet, daß Präzipitate in mehreren definierten Verdünnungen des zu bestimmenden Reaktionspartners gemessen werden.

3. Verfahren nach Anspruch 1, dadurch gekennzeichnet, daß in der Reaktion einer der Partner teils in gelöster Form, teils trägergebunden und der andere gelöst vorliegen.

4. Verfahren nach Anspruch 1, dadurch gekennzeichnet, daß man sich die Präzipitate in Gegenwart einer oder mehrerer aktiver Komplementkomponenten bilden läßt.

5. Verfahren nach Anspruch 1, dadurch gekennzeichnet, daß in einem Gefäß mit konkav gebogenem Boden gemessen wird.

6. Optische Vorrichtung zur quantitativen Bestimmung der Fläche zweidimensionaler Objekte, in folgender Reihenfolge bestehend aus a) einer Lichtquelle, b) der beleuchtenden Linse, c) einer Lochblende, d) dem Objekt, e) der abbildenden Linse und f) dem Sensor, der aus mehreren linear angeordneten Detektoren besteht, wobei entweder das Objekt oder der Sensor senkrecht zur optischen Achse beweglich angeordnet ist, so daß beim Überfahren die gesamte Fläche erfaßt wird.

7. Verwendung einer optischen Vorrichtung nach Anspruch 6 zum Vermessen von Agglutinationsmustern, die als Folge einer Reaktion eines Antigens, Antikörpers und gegebenenfalls einer oder mehrerer aktiven Komplementkomponenten entstanden sind.

1/1

FIG.1

FIG.2

FIG.3